# EUROPEAN PATENT SPECIFICATION

(11) **EP 4 203 788 B1**
(45) Date of publication and mention of the grant of the patent: **09.10.2024**
(21) Application number: 21794016.2
(22) Date of filing: 26.08.2021
(51) Int. Cl.: A61B 5/1486

(54) **CONTINUOUS GLUCOSE SENSOR AND MOUNTING ASSEMBLY**
KONTINUIERLICHER GLUCOSESENSOR UND MONTAGEANORDNUNG
CAPTEUR DE GLUCOSE EN CONTINU ET ENSEMBLE DE MONTAGE

(30) Priority: 26.08.2020 US 202063070735 P; 28.08.2020 US 202063072050 P
(43) Date of publication of application: 05.07.2023
(73) Proprietor: Tingo Medical Ltd., 6521136 Tel Aviv (IL)
(72) Inventor: STRAM, Lior, 6521136 Tel Aviv (IL); YODFAT, Ofer, 7177659 Modi'in (IL)
(74) Representative: Cooley (UK) LLP
(86) International application number: PCT/IL2021/051052
(87) International publication number: WO 2022/044017

(56) References cited:
- US-A1- 2012 323 098
- US-A1- 2013 150 691
- US-A1- 2018 325 433

## Description

### FIELD OF THE DISCLOSURE

The present disclosure is to systems for continuous monitoring of glucose in a patient.

### BACKGROUND

Continuous glucose monitoring is important to individuals with diabetes, as they must determine when insulin is needed to reduce glucose levels or when additional glucose is needed to raise the level of glucose. A continuous glucose monitoring device (CGM) usually adheres to a patient's skin and transmits glucose reading to a remote monitoring device. The CGM can be incorporated in a closed loop system (artificial pancreas) in which, an insulin pump automatically administers and adjusts insulin delivery according to CGM transmitted glucose readings (closed loop system).

Glucose monitoring systems according to the state of the art are for instance described in US2013/0150691A1, US2018/0325433A1 and US2012/0323098A1.

There exists a need for a fully disposable sensing device for continuous monitoring of glucose. There is also a need for a planar sensor probe having a minimal surface area and maximal electrodes surface area. There is also a need for a mounting device which is easy to use, minimizes pain, and minimizes insertion trauma. There is also a need for a mounting device that is fully automatic in which insertion and retraction of introducer sharp is done by a button press with no patient's intervention in spring pre-loading. There is also a need for a one piece, disposable mounting device that is pre-assembled in the factory and includes the sterilized sensor probe and the non-sterilized components and thus, reducing mounting steps at each device replacement. There is also a need for a one piece disposable mounting device that is cheap for production in which the relatively expensive spring loaded mechanism is not sterilized.

### SUMMARY

According to the invention, there is provided a continuous glucose monitoring system according to claim 1.

The disclosure presents a fully disposable, one piece continuous glucose monitoring (CGM) device (sensing device), which includes a sensor and a sensor probe, and a fully disposable, one piece, mounting assembly/unit for mounting the CGM device (mounting assembly, both mounting unit and mounting assembly used interchangeably). The device and the mounting unit are pre-assembled in the factory to a one piece, disposable, mounting assembly. The mounting assembly is comprised of sealed compartment that includes a portion of the sensor and a non-sealed compartment that includes another portion of the sensor. The mounting assembly can be partially, and preferably, fully automatic, at a button press - the sensor is adhered to the skin, and the sensor probe is inserted into the subcutaneous tissue.

The sensor probe, in some embodiments, includes a first portion which is inserted in the subcutaneous tissue and a second portion adapted to be received in a sensor housing (together the sensor probe, the glucose sensor, and sensor housing comprising a sensing device, as noted above). The sensor housing includes an upper cover and a lower cover, the lower cover including an adhesive, in some embodiments, for adhering the sensor to the skin. Following use, the sensing device (i.e., the sensor housing and sensor probe), in some embodiments, is removed from the body and disposed.

In some embodiments, the sensor comprises an electronic printed circuit board assembly ("PCBA" or "electronics") and may also include a variety of optional components, such as, for example, a receiver, a transmitter, a processing circuit, a battery, an alarm system, and/or a data storage unit. In some embodiments, the sensor includes a plurality of conductive contacts, e.g., two or more conductive contacts (e.g., conductive springs), that can be configured for coupling to two or more respective contact pads on the sensor probe. The sensor probe, in some embodiments, includes at least one working electrode, a counter electrode, at least two (2) electrical conductors, and at least two (2) contact pads. In some embodiments, the probe is planar and includes two (2) electrodes - a working electrode which can be positioned on one side of the probe, in some embodiments, at the distal end of the probe, and a counter electrode positioned, in some embodiments, on the opposite side. Both electrodes, in some embodiments, are connected with electrical conductors to contact pads that can be positioned on both sides of a contact(s) plate(s). The contact plate, in some embodiments, is perpendicular to the probe (and in some embodiments, can be a part of the probe) and can reside within the sensor housing such that at least one (1) contact pad is facing the PCBA, and another (e.g., the other) contact pad is facing the opposite direction. In some embodiments, the contacts plate is folded on one (1) side at 180 degrees such that both contact pads are facing the PCBA and thus, simplifying electrical connection. In some embodiments, the probe and the contact(s) plate(s) are made from a single matrix sheet (e.g. polyimide) folded such that the probe is configured to be perpendicular to the skin, the contacts plate is perpendicular to the probe (parallel to skin), and both contact pads are facing the PCBA.

A mounting unit is provided and configured for mounting the sensing device (e.g., see above embodiments) onto a patient. The mounting unit can be pre-assembled with the sensing device such that the mounting unit and the sensing device (which includes the sensor and the sensor probe) are provided in one piece (mounting assembly) that is packed in one box. The mounting unit, in some embodiments, is comprised of two compartments having two pre-assembled housings, a non-sealed, non-sterilized compartment (spring compartment) that includes the insertion and retraction mechanisms and a portion of the sensing device, and a sealed, sterilized compartment (probe compartment) that includes the sensor probe. Both compartments have housings, a spring compartment housing and a probe compartment housing, that are rigidly pre-assembled during manufacture (for example), the spring compartment housing on top of the probe compartment housing. The probe compartment is preferably sealed and sterilized with radiation (e.g., gamma or e-beam). Before device mounting, the patient removes a protecting lid from the bottom side of the sensor probe housing and adheres the mounting assembly to the skin. During device mounting, the sensor and sensor probe are displaced within the sensor probe housing and mounted onto the patient (with the probe being inserted into tissue).

An introducer for insertion of the sensor probe into the subcutaneous tissue is provided. The introducer, in some embodiments, includes a sharp tip at one end which is configured to penetrate the skin, and an introducer cap, at the other end, which is configured to drive the introducer in one direction during insertion and the opposite direction during retraction. The introducer, in some embodiments, is positioned within the mounting assembly and spans the non-sterilized compartment, the sterilized compartment, and an elastomeric septum (that provides sealing to the sealed, sterilized probe compartment). The introducer can be displaced through the septum in one direction during sensor probe insertion, and then, in an opposite direction during introducer retraction. Following device mounting, the mounting unit can be removed from the body leaving the sensor on the skin and the sensor probe within the subcutaneous tissue.

In some embodiments, after triggering, operation of the mounting unit is automatic. For example, upon pressing an operating button, the mounting unit adheres the sensor to the patient's skin, inserts the sensor probe into the subcutaneous tissue (mounting phase one) and retracts the sensor probe introducer while the sensor probe remains within the body (mounting phase two). After device mounting, the mounting unit can be removed from the body and disposed. In some embodiments, the mounting unit includes a spring loaded driving mechanism for mounting the device onto the patient, and a spring loaded retraction mechanism for removing the introducer while leaving the sensor on the patient. Upon pressing an operating button, a trigger releases a pre-loaded insertion spring that drives the device (including the sensor, sensor probe, and introducer) in a first direction. At the end of movement, a preloaded retraction spring is released, and the introducer is retracted in a second opposite direction such that the introducer sharp is concealed within the mounting unit. In some embodiments, the introducer is a rigid planar structure having a sharp tip and adapted to support the sensor probe during insertion. The introducer and sensor probe can concomitantly be inserted into the subcutaneous tissue; however, in some embodiments, at the end of movement of the introducer at first direction, the sensor probe is further advanced (e.g., for additional 1-3mm) in the same first direction. Thus, in such embodiments, local trauma within the surrounding tissue can be minimized because the cross profile of the sensor probe is very low (e.g., causes minimal trauma) and the glucose sensing electrode (i.e., the working electrode), that is located at the distal end of the probe, is exposed to a minimal local inflammatory reaction.

An assembly process for the device, according to some examples, includes the following steps (which in some examples are consecutive steps):
- deposition of glucose sensitive layers, electrical conductors, insulators, and contact pads on one or both sides of a matrix sheet;
- cutting and/or folding the matrix sheet such that it is configured to include the sensor probe and the contacts plate; and
- assembly (and sterilization) of the probe compartment, and assembly (stacking bottom-up) of the sensor PCBA, sensor housing upper cover, retraction spring, introducer cap and finally, the spring's compartment housing that includes the spring loaded insertion mechanism.

According to some examples, a method of mounting the device is provided, which includes the following steps (in some examples, consecutive steps): removal of the protecting lid, placement of mounting assembly on the skin, button press, and removal of mounting unit.

A continuous glucose monitoring system for continuously monitoring glucose levels of a user is provided. The system includes a mounting assembly, and a sensing device, where at least a portion of the sensing device is housed within the mounting assembly prior to mounting the sensing device on the user.

The above noted examples include one and/or another of the following additional features, functionality, structure, steps, or clarifications, yielding yet further embodiments (as is clear from the listing below, some of the additional features, functionality, structure, steps, and clarifications - as may be the case - build off of, and/or are based on, previously/earlier recited additional features, functionality, structure, steps, or clarifications):
- the sensing device can include at least a transmitter configured to transmit signals corresponding to sensed glucose levels of the user;
- a remote display unit configured with at least with a receiver for receiving the sensed glucose readings from the sensing device via the transmitter;
   o the sensor can include a lower portion (e.g., a lower cover) having an adhesive for removable affixation of the sensor to the skin of the user;
   o the sensor can include a sensor housing;
   o the sensor can include a sensor control unit;
   o the sensor probe can include a first portion configured for insertion into subcutaneous tissue, and a second portion configured for receiving by the sensor housing; and/or
   o the sensor probe can include a first portion configured for insertion into subcutaneous tissue, and a second portion configured for receiving by the sensor housing;
- the sensor housing can comprise an upper cover and a lower cover; where the lower cover can include an adhesive for adhering the sensor to the skin of the patient;
- the sensor can include a printed circuit board assembly (PCBA);
- at least one of the sensor housing and the PCBA can also include at least one, a plurality of, a majority of, substantially all of, or all of, a receiver, a transmitter, a processing circuit, a battery, an alarm system, and data storage means, where the receiver and the transmitter can together comprise a transceiver;
- the sensor can include at least one, and preferably, a plurality of conductive contacts configured for coupling to one or more respective contact pads of the sensor probe;
- the sensor probe can include:
   o at least one of: at least one working electrode, a counter electrode, and at least two (2) electrical conductors, and at least two (2) contact pads; or
   o includes at least one working electrode, a counter electrode, and at least two (2) electrical conductors, and at least two (2) contact pads;
- the sensor probe can be configured with a planar shape, and includes at least two (2) electrodes, where the at least two (2) electrodes comprises at least a working electrode;
- the working electrode can be configured for positioning on at least one of a first side of the sensor probe and at a distal end thereof;
- the at least two (2) electrodes can comprise a counter electrode;
- the at least two (2) electrodes can comprise at least a working electrode and a counter electrode, where the working electrode is configured for positioning on at least one of a first side of the sensor probe and at a distal end thereof, and the counter electrode is configured for arrangement on a side of the sensor probe opposite to the side where the working electrode is positioned on the sensor probe;
- each electrode can be connected via electrical conductors to a respective contact pad;
- one or more contact pads can be configured:
   o for positioning on at least one side of a contacts plate, the contacts plate optionally included with the sensor probe; or
   o for positioning on each side of a contacts plate, the contacts plate optionally included with the sensor probe;
- the contacts plate can be:
   o positioned approximately perpendicular to the sensor probe; and/or
   o positioned within the sensor housing;
- upon the contacts plate being positioned within the sensor housing (see above), at least one (1) contact pad can be facing the PCBA, and optionally, another (e.g., the other) contact pad can be facing an opposite direction;
- the contacts plate can be folded on a first side at 180 degrees, such that each contact pad is facing the PCBA (see above);
- the sensor probe and the contacts plates can be constructed from a matrix sheet configured such that the sensor probe is perpendicular to the skin, the contacts plate is perpendicular to the sensor probe (parallel to skin), and both contact pads are facing the PCBA;
- the matrix sheet can comprise a single matrix sheet;
- the mounting assembly can be assembled with the sensing device during manufacture, such that it is ready to mount the sensing device onto a patient;
- at least one of the first and second compartments can each include a housing;
- the first compartment can be arranged immediately adj acent the second compartment;
- the first compartment can include an insertion means and a retraction means;
- the second compartment can include the sensor probe and a portion of the sensor, and optionally an elastomeric septum configured to sealing the second compartment;
- the second compartment can include a protection lid;
- a side of the second compartment from which the protection lid is removed can be configured for placement adjacent to the skin of the patient;
- upon mounting the sensing device, at least one of the sensor and sensor probe can be displaced within the second compartment and mounted onto the patient;
- upon mounting the sensing device, the sensor and sensor probe can be displaced within the second compartment and mounted onto the patient;
- a sensor-probe introducer ("introducer") configured to insert the sensor probe into the subcutaneous tissue of the patient;
- the introducer can comprise at least one of, a plurality of, a majority of, substantially all of, or preferably all of:
   o a sharp tip at a first end configured to penetrate the skin of the patient, and
   o an introducer cap, at a second end configured to drive the introducer in a first direction during insertion and a second direction opposite the first direction during retraction;
      and
- the introducer:
   o can be positioned within the mounting assembly;
   o can be positioned within the mounting assembly and spans the first compartment, the second compartment, and the elastomeric septum;
   o can be configured for displacement through the septum in a first direction during sensor probe insertion, and in a second direction opposite to the first direction during introducer retraction;
   o can comprise a rigid planar structure including a/the sharp tip, and is adapted to support the sensor probe during insertion;
   o with the sensor probe, can concomitantly be inserted into the subcutaneous tissue;
   o can be planar in cross-section;
   o can be not oval or round in cross-section;
   o can include a u-shaped cross-section; and/or
   o can include at least one and preferably two shoulders configured to at least one of secure the sensor probe in insertion and allow introducer retraction, while leaving the sensor probe in the body;
- the mounting assembly can be configured to automatically operate, including automatically adhering the sensor to the patient's skin, inserting the sensor probe into the subcutaneous tissue, and retracting the sensor probe introducer;
- a trigger configured to initiate the automatic operation of the mounting assembly to mount the sensing device on the user;
- the trigger can include an operating button or an operating switch configured to receive user or operator input;
- the retraction means can comprise a spring loaded retraction mechanism for removing the introducer while leaving the sensing device on the patient;
- upon pressing a/the operating button, the trigger can be configured to release the insertion spring of the insertion means that drives the sensing device, including the sensor, the sensor probe, and optionally the introducer, in a first direction, thereafter, the retraction spring can be configured to release and the introducer can be retracted in a second opposite direction such that the sharp end of the introducer can be at least substantially concealed within the mounting unit;
- at the end of insertion, the sensor probe can be further advanced in the first direction (e.g., 1-3 mm);
- the second compartment (i.e., probe compartment) can be sterilized first with an e-beam and/or gamma radiation.

In some examples, 2. an assembly method for assembling a mounting assembly and a sensing device for a continuous glucose monitoring system is provided and includes assembling a/the sensing device and assembling a/the mounting assembly. For assembling the sensing device, the method includes at least one of, and preferably a plurality of, and most preferably all of:
- depositing glucose sensitive layers, electrical conductors, insulators, and contact pads on one or both sides of a matrix sheet, such depositing includes forming one or more electrodes on the matrix sheet;
- cutting and/or folding the matrix sheet such that it is configured to include the sensor probe and the contacts plate; and
- assembling of the sensor contacts plate, sensor printed-circuit-board-assembly (PCBA), and sensor housing as to form a sensor

For assembling of the mounting assembly, the method includes at least one of, and preferable a plurality of, and most preferably all of:
- assembling at least one of an insertion spring and a retraction spring into a first compartment of the mounting assembly;
- assembling and sterilizing a second compartment of the mounting assembly; and
- placing at least a portion of the sensing device in the second compartment.

Such examples, 2. may also include one and/or another of the following additional features, functionality, structure, steps, or clarifications, yielding yet further embodiments (as is clear from the listing, some of the additional features, functionality, structure, steps, and clarifications - as may be the case - build off of, and/or are based on, previously/earlier recited additional features, functionality, structure, steps, or clarifications):
- the second compartment is sterilized first with an e-beam and/or gamma radiation;
- at least one of the probe and contacts plate are formed on/from the matrix sheet;
- the sensor probe and/or the contacts plate can be formed on/from the matrix sheet;
- at least one of the sensor probe and the contacts plate can be formed on/from the matrix sheet via cutting and/or folding so as to form an/the appropriate spatial configuration;
- at least one of the one or more electrodes, one or more conductors, and one or more contact pads, can be positioned on at least one side of the matrix sheet;
- at least one of the one or more electrodes, one or more conductors, and one or more contact pads, can be positioned on both sides of one matrix sheet;
   and
- the folding of contacts plate can be configured to arrange the contact pads to face the same direction, where the same direction can be towards the PCBA.

These and other advantages, and objects thereof, of the present disclosure are even more evident given the detailed description of at least some of the examples which follow, as well as the figures that form part of this disclosure (a brief description of which is outlined below).

### BRIEF DESCRIPTION OF THE DRAWINGS

**Figure 1** shows a scheme of the CGM system that includes a mounting assembly and the display unit.
Figures 2A-C show cross-sectional view (2A), spatial/perspective (2B), and oblique cross section (2C) views of a mounting assembly.
Figures 3A-B show exploded (3A) and cross-section (3B) views of a probe compartment.
**Figure 4** shows a cross-sectional view of a mounting assembly after removal of a protecting lid.
**Figure 5** shows a cross-sectional view of a mounting assembly at the end of a first mounting phase.
**Figure 6** shows a cross-sectional view of a mounting assembly at the end of a second mounting phase.
Figures 7A-B shows a spatial view (7A) and a cross-sectional view (7B) of a mounting assembly after removal from a sensor.
Figures 8A-B show a spatial view (8A) and a cross-sectional view (8B) of a sensor.
**Figure 8C** shows a cross section view of a sensor on a patient.
**Figure 9A** shows a cross section view of a spring release mechanism.
**Figures 9B** and **9C** are magnified views of a release mechanism of an insertion spring (9B) and a retraction spring (9C), respectively.
**Figure 10** shows an exploded view of parts of a sensor.
**Figure 11** shows a sensor. 2.
**Figure 11A** shows a top level view of a sensor 50.
**Figure 11B** shows a spatial, exploded view of a sensor.
**Figure 11C** shows a cross-sectional view of a septum and an introducer configured to cross the septum.
**Figure 12** shows parts of a sensor.
**Figure 12A** shows a spatial view of a sensor printed circuit board (PCBA), a sensor base plate, and an introducer.
**Figure 12B** shows a spatial view of an introducer, a contacts plate, and a conductive spring.
**Figure 12C** shows an exploded view of an introducer, a sensor probe, a contacts plate, conductive springs, and conductive spring holders.
**Figures 13A****-A1** show a sensor probe and a contacts plate after (13A1) and before (13A) folding of a matrix sheet.
Figures 13B - B1 show spatial views of mirror images of a sensor probe and a contacts plate, electrodes, conductors, and contact pads.
**Figure 14** show spatial views of an introducer, a probe and a contacts plate.
**Figure14A** shows an exploded view of an introducer and a probe before sensor probe insertion.
Figures 14B-C show an introducer and a sensor probe before insertion, with Figure 14B showing a magnified view of an introducer tip and a probe tip before sensor probe insertion, and Figure 14C showing a magnified view of the introducer tip and the probe tip after probe insertion (before introducer retraction).
Figures 15A1-3 show a side view (15A1), a top view (15A2), and a cross-sectional view (15A3) of an introducer tip.
Figures 15B1-2 show a side view (15B1) and a top view (15B2) of an introducer tip and a sensor probe tip.
Figures 15C and 15D1-2 show top views of some preferred embodiments of sensor probe tip 65.
Figures 16A - C and 16A1 - C1 show spatial and magnified views, respectively, of a sensor, a sensor probe, an introducer tip, and a probe tip, during first and second mounting phases (i.e., insertion of introducer and sensor probe, and retraction of introducer, respectively).

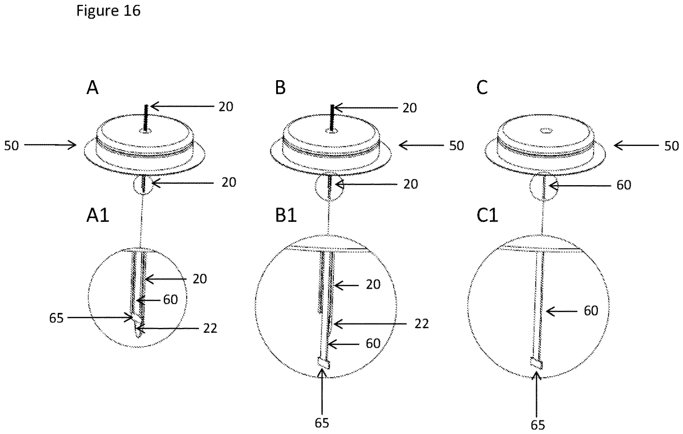

Figures 16A - A1 show a sensor probe coupled with an introducer.
Figures 16B - B1 show a probe tip protruding from an introducer tip.
**Figures 16C****- C1** show a sensor and a probe after removal of an introducer.

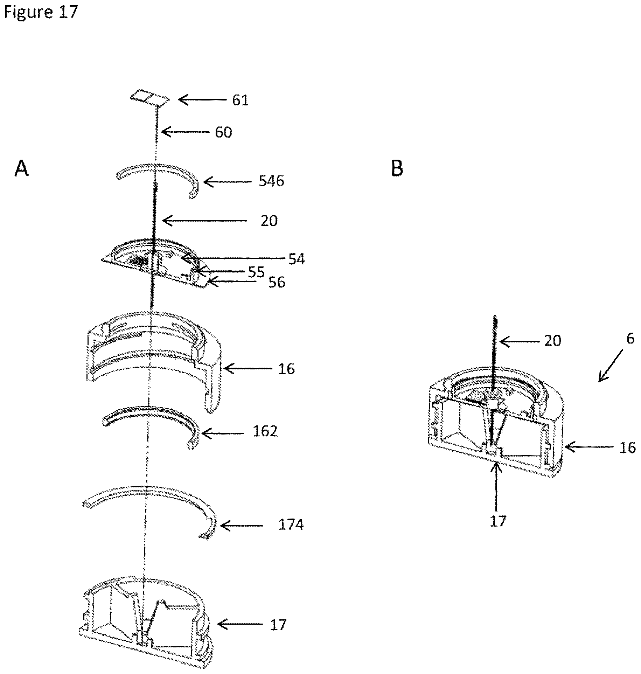

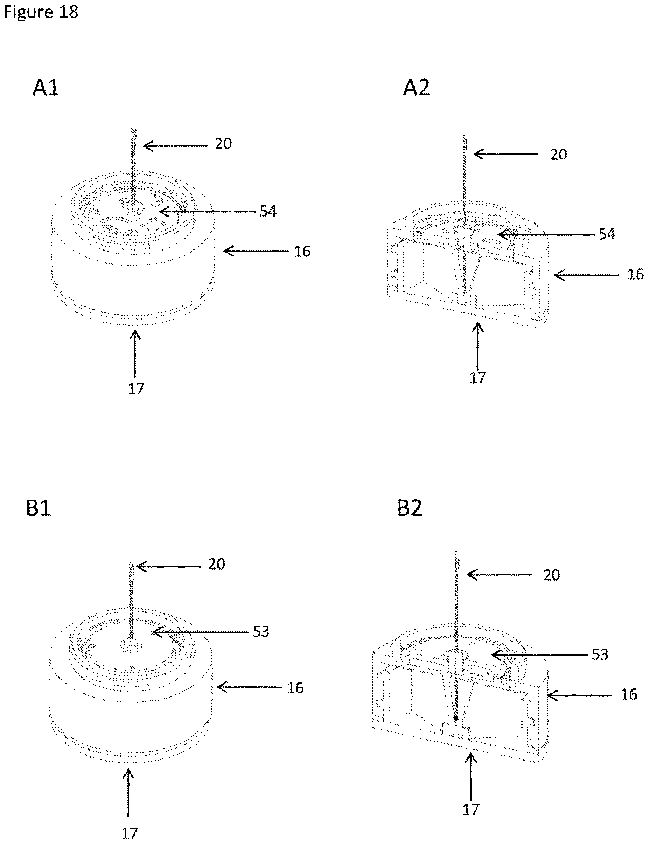

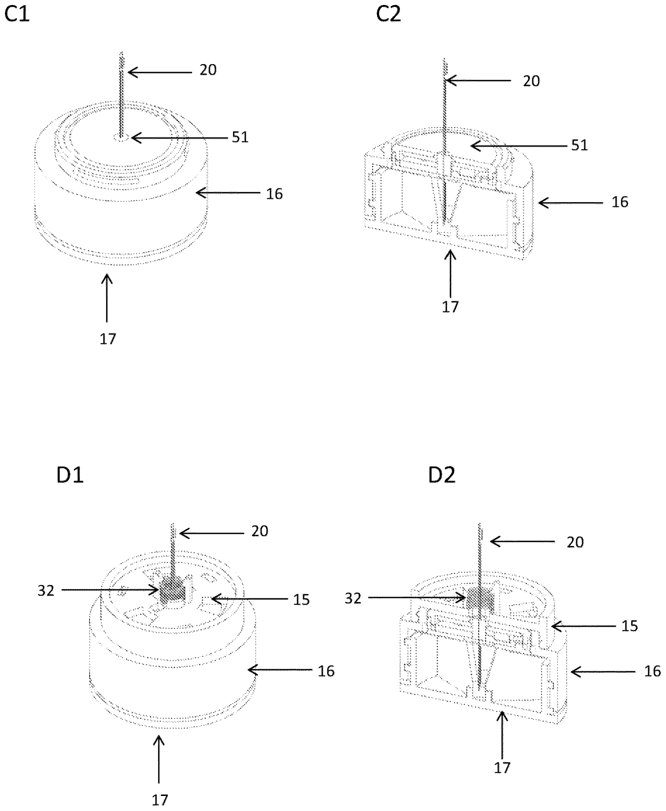

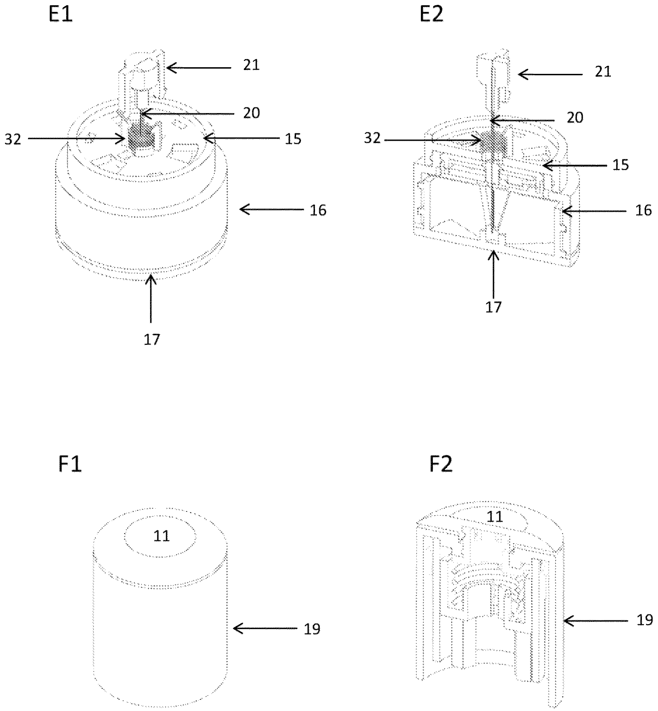

**Figures 17** and **18** show an assembly process of a mounting assembly.
Figures 17A-B show exploded view (17A) and oblique cross-sectional view (17B) of a sterilized, sealed compartment (a second/probe compartment).
Figures 18A1 - F1 and 18A2 - F2 show spatial views (18A1-F1) and oblique cross-sectional views (18A2 - F2), respectively, of consecutive assembly stages of a mounting assembly.

### DETAILED DESCRIPTION

The following reference numbers and associated terms are used to describe various structures of the present disclosure.
- 1: Mounting assembly
- 2: Display unit
- 5: matrix sheet (after folding forms the sensor probe and contacts plat)
- 6: Probe compartment (in some embodiments, referred to as a/the second compartment)
- 7: First electrode (working)
- 8: Second electrode (counter)
- 9: Spring compartment
- 10: Mounting unit
- 11: Operating button
- 12: Trigger
- 13: Sleeve
- 14: Hammer
- 141: Hammer introducer pulling lever
- 142: Hammer sensor pushing lever
- 15: Spring compartment base
- 155: Retraction spring latch
- 16: Probe compartment housing
- 161: Probe compartment housing worm
- 162: Probe compartment seal
- 17: Protecting lid
- 171: Protecting lid supporting ribs
- 172: Protecting lid thread
- 173: Protecting lid base
- 174: Protecting lid seal
- 18: Trigger latches
- 19: Spring compartment housing
- 20: Introducer
- 21: Introducer cap
- 211: Introducer cap snap
- 212: Introducer cap snap holder
- 213: Introducer cap knob
- 22: Introducer tip
- 23: Introducer probe holder
- 31: Insertion spring
- 32: Retraction spring
- 50: Sensor
- 51: Sensor housing upper cover
- 52: Sensor septum
- 53: Sensor printed circuit board assembly (PCBA)
- 533: print opening
- 54: Sensor base plate
- 541: Base plate opening
- 542: Conductive spring
- 543: Conductive spring holder
- 544: Base plate battery groove
- 546: Base plate seal
- 55: Sensor housing lower cover
- 551: Lower cover cushion pad
- 56: Sensor adhesive
- 60: Sensor probe
- 65: Sensor probe tip
- 61: Contacts plate
- 611: Contacts plate first side
- 7: First electrode (working)
- 71: Conductor first electrode (working)
- 72: Contact pad first electrode (working)
- 612: Contacts plate second side
- 8: Second electrode (counter)
- 81: Conductor second side (counter)
- 82: Contact pad second electrode (counter)
- 613: Contacts plate fold
- 70: Subcutaneous tissue
- 80: Skin

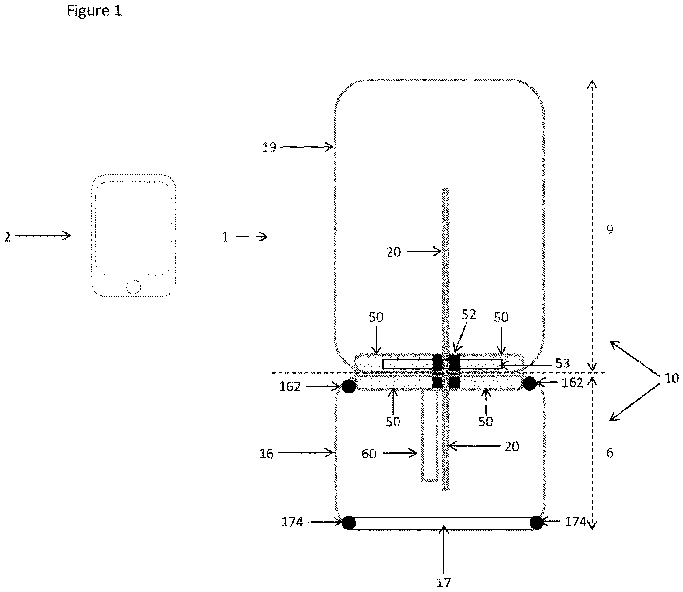

**Figure 1** shows a scheme of a continuous-glucose-monitoring (CGM) system, according to some embodiments, that includes an implantable subcutaneous glucose sensitive probe for detecting glucose levels in the interstitial fluid of a patient. The CGM system, in some embodiments, includes a disposable mounting assembly 1 and a durable remote display unit 2. Remote display unit 2, in some embodiments, has a receiver for receiving glucose readings from a skin adhered glucose sensor ("sensor" or "glucose sensor" 50), and may also include a screen for presenting the received glucose readings. Mounting assembly 1, according to some embodiments, is a one-piece, disposable unit that includes sensor 50 and a mounting unit 10 for mounting sensor 50 onto a patient. Mounting unit 10 and sensor 50, in some embodiments, are pre-assembled during manufacture, and thus, are provided in one single disposable unit - mounting assembly 1. Before sensor mounting (on the body), sensor 50, in some embodiments, at least a portion of the sensor is concealed, and in some embodiments, substantially all or all of the sensor is concealed, within the mounting unit 10.

Mounting assembly 1 is comprised of two (2) compartments: a non-sterilized, non-sealed compartment, which may also be referred to as a first or spring compartment 9, and a second, sterilized, sealed compartment, which may also be referred to as a probe compartment 6. The sensor, in some embodiments, comprises at least two (2) components that can be pre-assembled during manufacture: a sensor control unit, which in some embodiments, includes a housing adapted for placement on the skin, and a sensor probe 60 that has a first portion configured for insertion in the subcutaneous tissue and a second portion configured for being received in a sensor housing. Sensor probe 60, in some embodiments, includes a glucose sensitive enzyme configured for detecting glucose levels in the interstitial fluid within the subcutaneous tissue. Sensor 50 is positioned within mounting assembly 10 such that at least a first portion of sensor 50 can be received in the sealed, sterilized compartment (second/probe compartment 6), and at least another portion (e.g., second portion) of sensor 50, which includes a PCBA 53, which is adopted to be received in the non-sealed, non-sterilized compartment (spring compartment 9).

In some examples, which are not claimed, both compartments of mounting assembly 1 are sterilized. In this configuration, second/probe compartment 6 is sterilized with, for example, an e-beam or gamma radiation, and spring compartment 9 is sterilized with a gas (e.g., ethylene oxide). The portion of sensor 50 which includes PCBA 53 is configured to be received in a sterilized compartment (spring compartment 9).

An introducer 20 is included, which is configured for inserting sensor probe 60 into subcutaneous tissue. Introducer 20, in such embodiments, may be positioned within mounting assembly 1, and can span non-sterilized spring compartment 9, sterilized probe compartment 6, and an elastomeric septum 52. Probe compartment 6 can include a housing 16, a removable lid 17, a portion of sensor 50, and a portion of introducer 20. Sealing can be provided by a protecting lid seal 174, probe compartment seal 162, and septum 52.

The spring compartment, in some embodiments, includes a housing 19, a portion of introducer 20, and spring mechanisms (not shown) for driving introducer 20 in a first direction (e.g., probe 60 insertion), and a second direction, opposite to the first direction (e.g., introducer 20 retraction). In some embodiments, before use, a patient removes protecting lid 17 providing a free forward movement (e.g., in **Figure 1** - downward movement) of sensor 50 and sensor probe 60 within probe compartment housing 16.

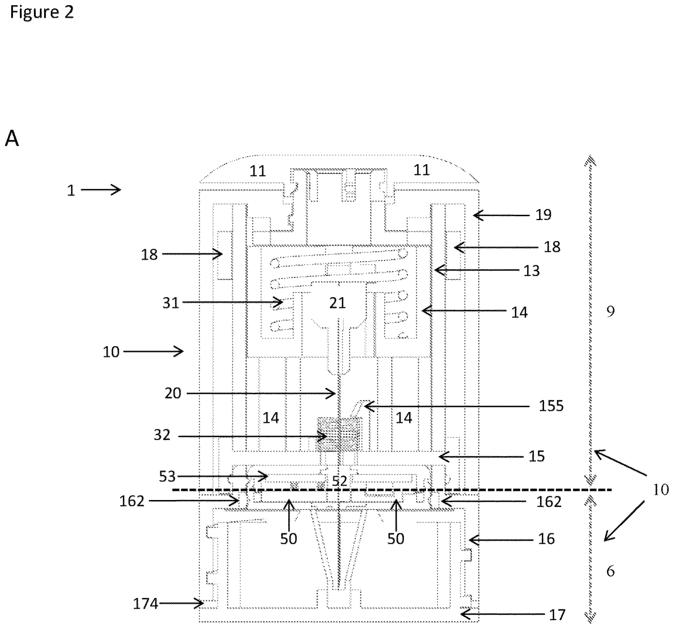

Figures 2A-C show cross-sectional **(2A)**, spatial **(2B)**, and oblique cross-sectional **(2C)** views of mounting assembly 1 that, in some embodiments, is comprised of mounting unit 10 and sensor 50. **Figure 2A** shows mounting unit 10 that can be comprised of probe compartment 6 and spring compartment 9. Probe compartment 6, spring compartment 9, and sensor 50, in some embodiments, can be pre-assembled during manufacture, forming mounting assembly 1. The probe compartment can include probe compartment housing 16, removable protecting lid 17, a portion of sensor 50, sensor probe (not shown), and at least a portion of introducer 20. Sealing of probe compartment 6 can be provided by protecting lid seal 174, probe compartment seal 162, and septum 52. Spring compartment 9 can include a spring compartment housing 19, a portion of introducer 20 including introducer cap 21, and one or more spring mechanisms (e.g., insertion and retraction spring loaded mechanisms).

The spring mechanisms, in some embodiments, include at least a plurality of (and in some embodiments, all of) an operating button 11, a trigger 12, trigger latches 18, a sleeve 13, hammer 14, insertion spring 31, retraction spring 32, and retraction spring latch 155. Accordingly, following a press of operating button 11, trigger 12 releases hammer 14, insertion spring 31 drives hammer 14 in a first direction (e.g., forward) which drives introducer cap 21, introducer 20, and sensor 50 in the same direction. When introducer cap 21 reaches the end of the forward movement (e.g., end of a first mounting phase), in some embodiments, it releases retraction spring latch 155 and retraction spring 32 drives introducer cap 21 and introducer 20 in a second direction, opposite to the first direction (e.g., backward) direction.

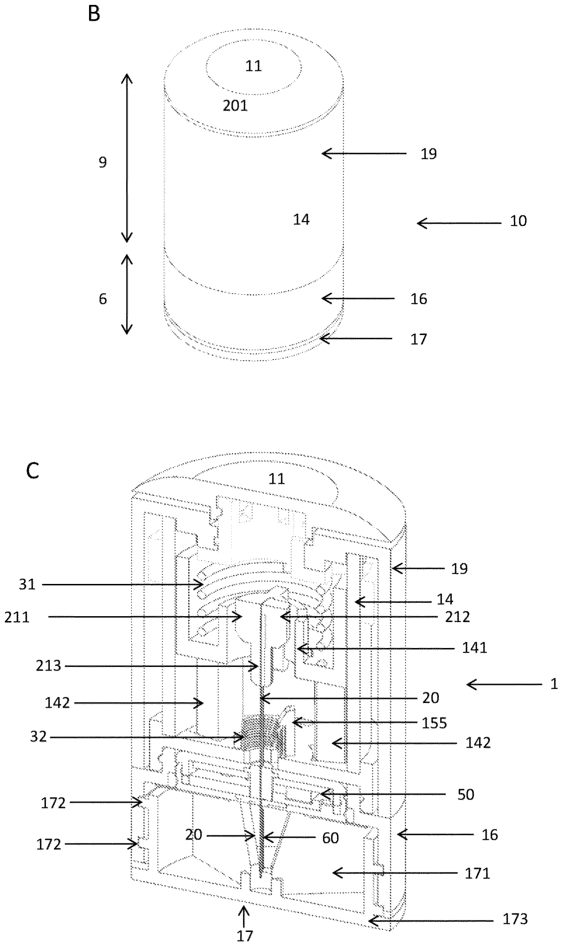

**Figure 2B** shows a spatial view of one-piece mounting unit 10, including probe compartment 6, probe compartment housing 16, spring compartment 9, spring compartment housing 19, and operating button 11. **Figure 2C** shows an oblique cross-sectional view of mounting assembly 1, according to some embodiments, that includes probe compartment housing 16, spring compartment housing 19, sensor 50, sensor probe 60, and introducer 20. Removable protecting lid 17 can include a protecting lid base 173, and can be connected to probe compartment housing 16 with a protecting lid thread 172. In some embodiments, protecting lid supporting ribs 171 provide support to the probe compartment and sensor probe protection. Introducer 20 can be connected at one end (e.g., proximal end) to an introducer cap 21, that, in some embodiments, is comprised of introducer cap snap 211, introducer cap snap holder 212, and introducer knob 213.

In some embodiments, the insertion mechanism includes operating button 11, trigger 12, sleeve 13, hammer 14, and insertion spring 31. The hammer 14 can include the introducer pulling lever 141, and sensor pushing lever 142 (e.g., three (3) levers). In some embodiments, the release mechanism includes retraction spring 32 and retraction spring latch 155. In some embodiments, before use, the protecting lid is removed by patient, and upon pressing of operating button 11, trigger 12 releases the hammer 14 and insertion spring 31 drives hammer 14 in a (first) forward direction within sleeve 13. In some embodiments, hammer introducer pulling lever 141, that is connected to introducer cap snap 211, drives introducer 20 in the (first) forward direction, and concomitantly (in some embodiments), the hammer sensor pushing levers 142 drive sensor 50 and sensor probe 60 also in the (first) forward direction. At the end of forward movement of hammer 14 (end of the first mounting phase), introducer cap knob 213 releases retraction spring 32 which drives introducer cap 21 in an opposite (second), e.g., backward, direction.

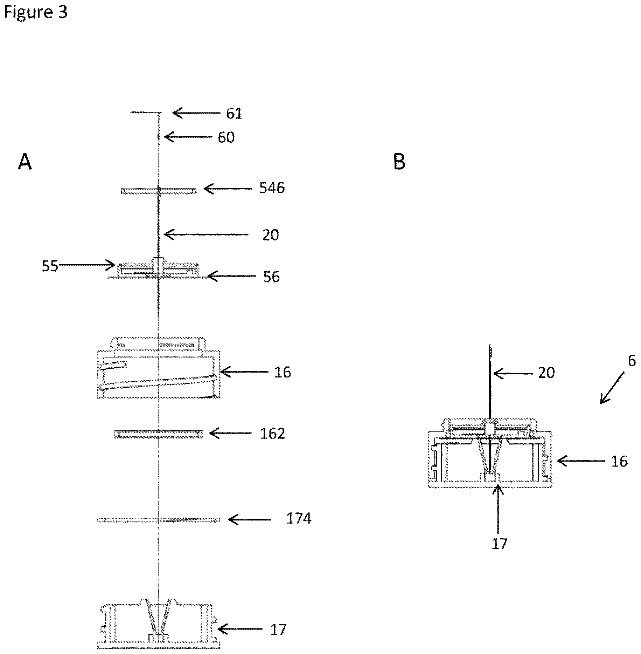

Figures 3A-B show exploded **(3A)** and cross-sectional **(3B)** views of the sealed, sterilized, probe compartment 6, according to some embodiments. **Figure 3A** shows parts of probe compartment 6 (stacked, e.g., from the bottom up): protecting removable lid 17, protecting lid seal 174, probe compartment seal 162, probe compartment housing 16, sensor housing lower cover 55 and adhesive 56, introducer 20, sensor base plate 54 (not shown), base plate seal 546, sensor probe 60, and contacts plate 61. Protecting lid seal 174 and probe compartment seal 174 can be included so as to provide sealing to the probe compartment 6 before mounting the sensor 50 to a patient's body. Sensor base plate seal 546 can be included to provide sealing to the sensor after sensor mounting while the sensor is adhered to the patient's skin. **Figure 3B** shows a cross-sectional view of an assembled probe compartment 6: sensor probe housing 16, introducer 20, and protective lid 17 (shown).

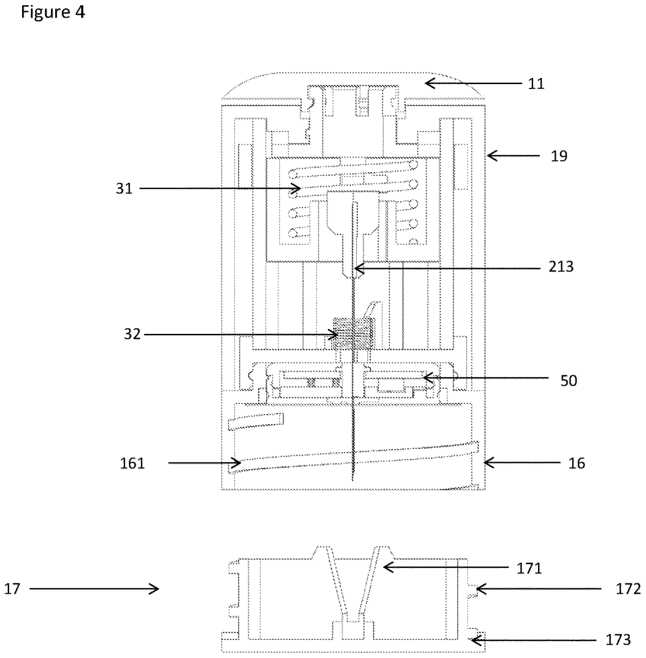

**Figure 4** shows a cross-sectional view of mounting assembly 1 after removal of protecting lid 17 (before sensor mounting), according to some embodiments. Accordingly, mounting assembly 1 can include spring compartment housing 19, sensor probe housing 16, insertion spring 31, retraction spring 32, and introducer cap knob 213. Protecting lid 17, can include protecting lid base 173, protecting lid thread 172, and protecting lid ribs 171. Before sensor 50 mounting, the protecting lid 17, in some embodiments, is removed by the patient by unscrewing the thread 172 from the probe compartment housing worm 161.

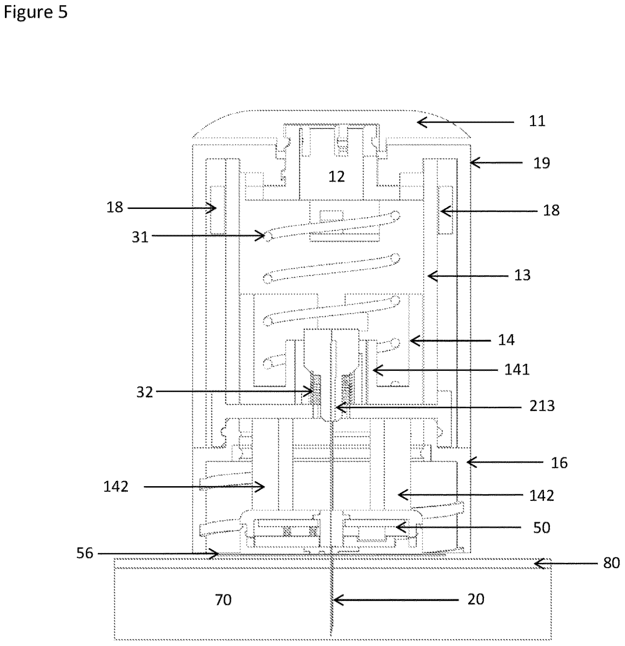

**Figure 5** shows a cross-sectional view of mounting assembly 1 at the end of hammer 14 forward (first direction) movement (end of the first mounting phase). Accordingly, sensor 50 is adhered to the skin 80 with adhesive 56 and introducer 20 and sensor probe (not shown) are inserted within the subcutaneous tissue 70. Mounting assembly 1, in such embodiments, can include a plurality of, and in some embodiments, all of, spring compartment housing 19, probe compartment housing 16, operating button 11, trigger 12, trigger latches 18, sleeve 13, hammer 14, hammer sensor pushing levers 142, insertion spring 31, retraction spring 32, introducer 20, and introducer knob 213. Following the pressing of operating button 11, trigger 12 releases the hammer 14 and insertion spring 31 drives hammer 14 in a forward (first) direction within the sleeve 13. In some embodiments, at the end of forward movement of hammer 14, introducer knob 213 is positioned within the loaded retraction spring 32, sensor 50 is adhered to the skin 80, and introducer 20 and sensor probe 60 are inserted within the subcutaneous tissue 70.

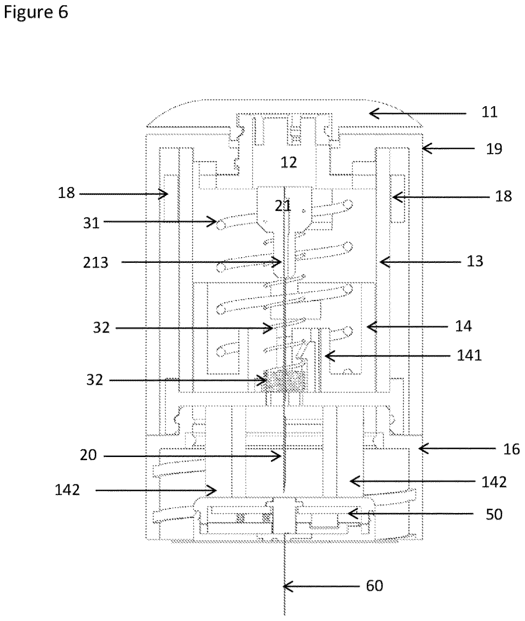

**Figure 6** shows a cross-sectional view of the mounting assembly at the end of introducer 20 retraction (end of a second mounting phase). The mounting assembly, in some embodiments, can include a plurality of, and in some embodiments, all of, probe compartment housing 16, spring compartment housing 19, operating button 11, trigger 12, sleeve 13, hammer 14, hammer introducer pulling lever 141, hammer sensor pushing levers 142, insertion spring 31, retraction spring 32, introducer 20, introducer cap 21, and introducer knob 213. Accordingly, in some embodiments, sensor 50 is adhered to skin (not shown) and sensor probe 60 is inserted within the subcutaneous tissue (not shown). Following introducer 20 insertion, (i.e., the end of forward/first movement of hammer 14), retraction spring 32 is released and the introducer cap 21, introducer knob 213, and introducer 20 are driving at the opposite direction (backward/second-direction movement). At the end of the second mounting phase, introducer 20 is concealed within mounting assembly 1.

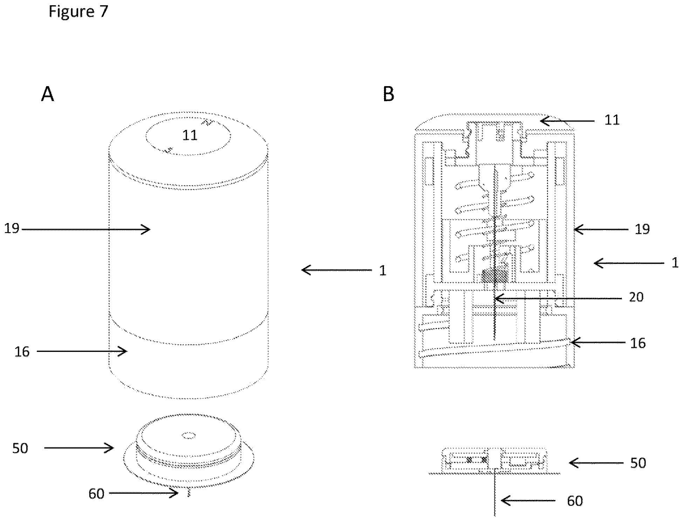

Figures 7A-B show spatial **(7A)** and cross-sectional **(7B)** views of mounting assembly 1 after removal from the body, sensor 50, in some embodiments, is mounted on the skin (not shown) and sensor probe 60 is inserted in the subcutaneous tissue (not shown). Mounting assembly 1 includes probe compartment housing 16, spring compartment housing 19, operating button 11, and introducer 20.

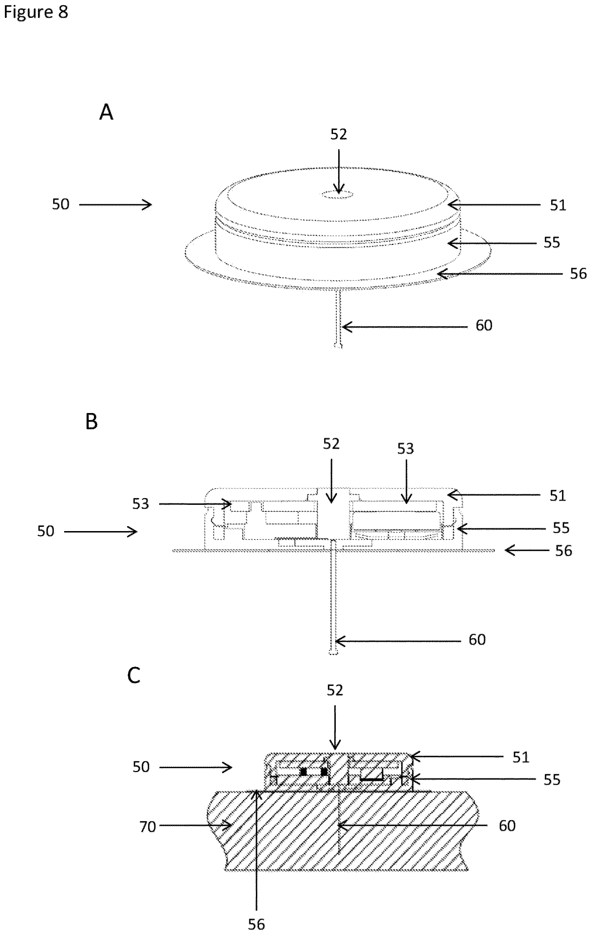

Figures 8A-B show spatial **(8A)** and cross-sectional **(8B)** views of sensor 50, according to some embodiments. **Figure 8A** shows sensor 50 and sensor probe 60, with sensor 50 including, in some embodiments, sensor housing upper cover 51, sensor housing lower cover 55, septum 52, and adhesive 56. **Figure 8B** shows the sensor housing upper cover 51, sensor housing lower cover 55, PCBA 53, septum 52, and adhesive 56. The sensor housing lower cover 55, sensor base plate 54 (not shown), septum 52, and adhesive 56 can be configured to be received in the sterilized compartment (e.g., probe compartment 6), and PCBA 53 and sensor housing upper cover 51 are configured to be received in the non-sterilized compartment (e.g., spring compartment 9). **Figure 8C** shows a cross section view of the sensor 50 on a patient skin, the sensor probe 60 is inserted within the subcutaneous tissue. The sensor 50 includes the sensor housing upper cover 51, sensor housing lower cover 55, septum 52, and adhesive 56.

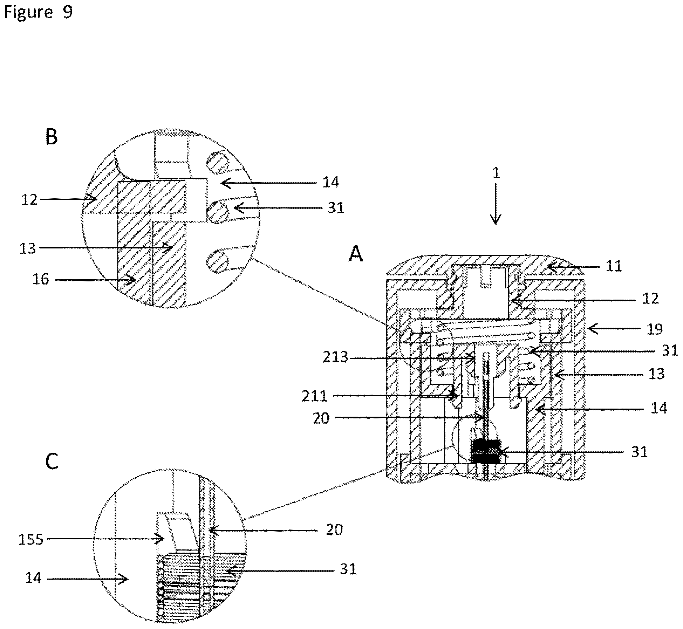

**Figure 9** shows the spring release mechanisms, according to some embodiments. **Figure 9A** shows a cross-sectional view (cross-section plan is 45° rotated vs. **Figure 2** cross section plan) of the spring release mechanisms. **Figures 9B** and **9C** are magnified views of the release mechanisms of insertion spring 31 **(9B)** and retraction spring 32 **(9C)**, respectively. **Figure 9A** shows mounting assembly 1 that includes at least a plurality of, and in some embodiments, all of: spring compartment housing 19, operating button 11, trigger 12, sleeve 13, hammer 14, insertion spring 31, retraction spring 32, introducer 20, introducer cap snap 211, and introducer cap knob 213. During insertion, and in some embodiments, hammer 14 is displaced in a forward (e.g., first) direction by loaded spring 31. The introducer cap snap 211 is displaced by hammer 14 in the same (forward/first) direction and concomitantly drives introducer 20 and introducer cap knob 213 in the same (forward/first) direction. **Figure 9B** shows the release mechanism of insertion spring 31, according to some embodiments, where upon pressing operating button 11, the trigger 12 is displaced in the same (forward/first) direction and rotates (in some embodiments, slightly, e.g., less than one rotation) such that hammer 14 is released and displaced (e.g., forward) within sleeve 13 by loaded spring 31.

**Figure 9C** shows the retraction mechanism of retraction spring 32, according to some embodiments. Here, retraction spring latch 155 is laterally displaced by the introducer cap knob 213, and releases the retraction spring 32. Retraction spring 32 is then displaces the introducer 20 in a second/backward direction opposite to the first/forward direction.

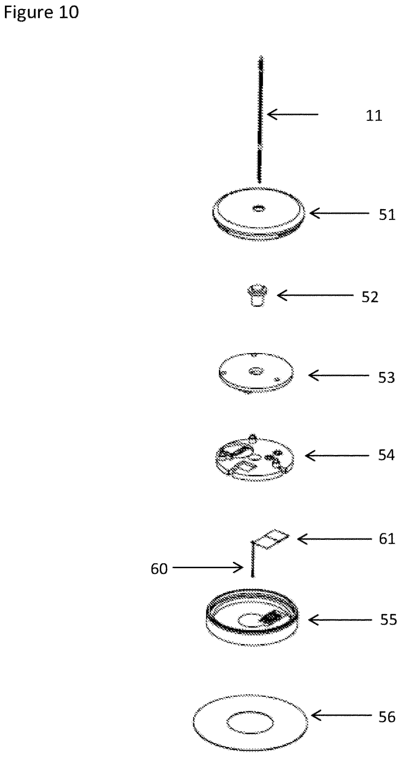

**Figure 10** shows an exploded view of parts/components of sensor 50 and sensor probe 60, according to some embodiments. Sensor 50 and sensor probe 60 can include (in a stacked arrangement, e.g., stacking bottom-up) at least a plurality of, and in some embodiments, all of: adhesive 56, sensor housing lower cover 55, sensor probe 60, contacts plate 61, sensor base plate 54, PCBA 53, septum 52, and sensor housing upper cover 51. The adhesive 56, sensor housing lower cover 55, sensor base plate 54, sensor probe 60, contacts plate 61, septum 52 and a portion of introducer 20 are sterilized (according to some embodiments); PCBA 53 and sensor housing upper cover 51 are not sterilized (according to some embodiments).

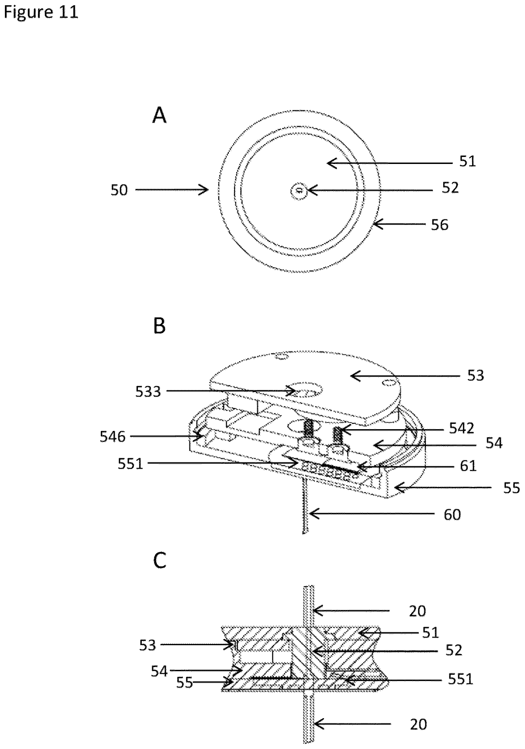

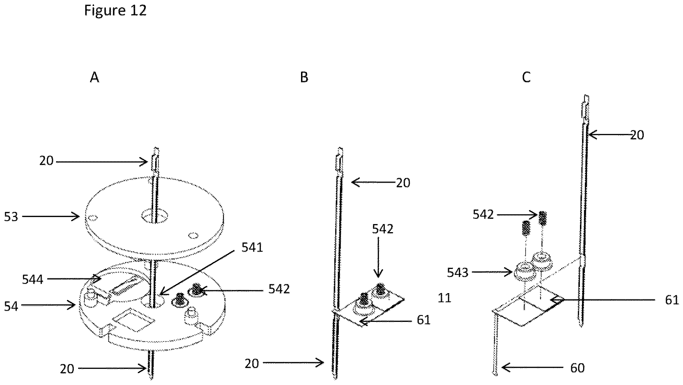

**Figure 11** shows sensor 50 components, according to some embodiments. **Figure 11A** shows a top level view of sensor 50, according to some embodiments, that includes adhesive 56, septum 52, and sensor housing upper cover 51. **Figure 11B** shows a spatial, exploded view of sensor 50 and sensor probe 60. Sensor 50 can include a plurality of, and in some embodiments, all of sensor housing lower cover 55, lower cover cushion pad 551, contacts plate 61, base plate seal 546, sensor base plate 54, conductive springs 542, PCBA 53, and PCBA opening 533. The base plate seal 546, in some embodiments, provides sealing (e.g., water resistance) for sensor 50 after connection of sensor housing lower cover 55 and sensor housing upper cover 51. The lower cover cushion pad 551, in some embodiments, can provide tight/rigid connection between contacts pad (shown in **Figures 12-14****)** that are located on contacts plate 61 and the conductive springs 542. In some embodiments, during sensor operation, electrical current generated on sensor probe 60 is conducted to contacts plate 61 and through conductive springs 542 to the PCBA 53. **Figure 11C** shows a cross-sectional view of a portion of sensor 50 that includes, according to some embodiments, sensor housing lower cover 55, sensor base plate 54, PCBA 53, and sensor housing upper cover 51. In some embodiments, introducer 20 crosses (e.g., through/in) septum 52.

**Figure 12** shows the sensor components/parts, according to some embodiments. **Figure 12A** shows a spatial view of the sensor electronic print (PCBA) 53, sensor base plate 54, and introducer 20. Sensor base plate 54 can include at least one of, and preferably both of, a recess 544 for housing the battery (not shown), base plate opening 541, and openings for the conductive springs 542. Introducer 20 can span the sensor through openings in PCBA 53 and sensor base plate 54 (septum 52 that crosses these openings is not shown). Figure 12B shows a spatial view of introducer 20, contacts plate 61, and conductive springs 542, according to some embodiments. Figure 12C shows an exploded view of introducer 20, sensor probe 60, contacts plate 61, conductive springs 542, and conductive spring holders 543 (sensor base plate 54 and PCBA 53 removed), according to some embodiments.

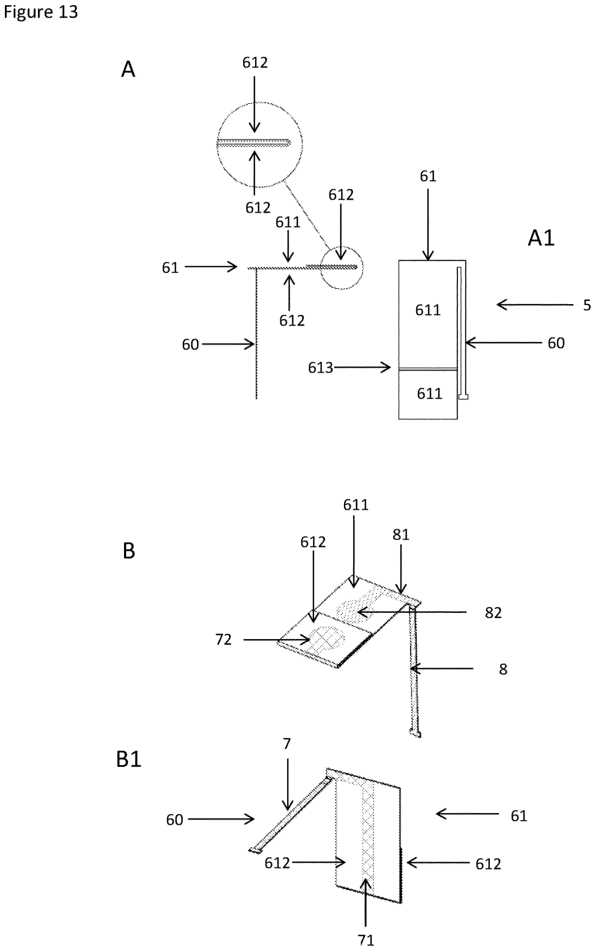

**Figure 13** shows sensor probe 60 and contacts plate 61, according to some embodiments. Sensor probe 60 and contacts plate 61 can be made from a single, flat thin base sheet (matrix sheet, e.g., polyimide). Accordingly, electrodes, conductors, insulators, contact pads, enzyme layer, and other protective layers can be deposited on one side or both sides thereof. Following deposition of materials on the matrix sheet, it is cut (e.g., dye cut or laser cut) to a desired shape and can be folded to receive a final spatial configuration such that the contacts plate 61 is adopted to be received in the sensor housing and the sensor probe (perpendicular to contacts plate) is adopted to be inserted in the subcutaneous tissue. In some embodiments, the contacts plate is configured to provide electrical contact between sensor probe electrodes and the sensor PCBA. The electrodes (at least one) can be deposited on one or both sides of the matrix sheet such that, for example, the working electrode (i.e., glucose sensitive) is deposited on one side and the counter electrode is deposited on the other/second side.

In some embodiments, the working electrode and the counter electrode (and, if required, a reference electrode) are deposited on one side of the matrix sheet. **Figure 13A** shows a side view of sensor probe 60 and contacts plate 61 after folding of the matrix sheet, according to some embodiments. Here, contacts plate 61 has 2 sides - contacts plate first side 611 and contacts plate second side 612. In some embodiments, the contacts plate 61 is folded such that contacts plate first side 611 and contacts plate second side 612 are facing the same direction (e.g., upside) (magnified view). Figure 13A1 shows a top view of the matrix sheet 5 after cutting and before folding, according to some embodiments. Here, matrix sheet 5 includes the sensor probe 60 and contacts palate 61 that is comprised of the contacts plate first side 611, the contacts plate second side 612 (not seen in a top view), and a contacts plate fold 613. Figures 13B and 13B1 show two spatial views (mirror images) of the folded matrix sheet that forms the sensor probe 60 and the contacts plate 61, according to some embodiments, and includes electrodes 7 and 8, conductors 71 and 81, and contact pads 72 and 82. Electrode 7, conductor 71, and contact pad 72 can be deposited on one side of the matrix sheet 5; the contact pad 72 can be deposited on the contacts plate second side 612. Electrode 8, conductor 8, and contact pad 82 can be deposited on the second side of the matrix sheet 5; the contact pad 82 can be deposited on contact plate first side 611. In some embodiments, after folding of matrix sheet, contact pad 72 and contact pad 82 face the same direction.

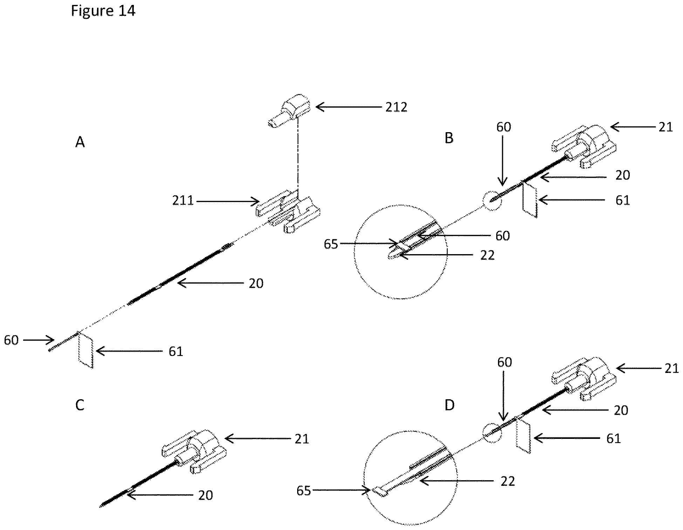

**Figure 14** show spatial views of introducer 20, probe 60, and contacts plate 61, according to some embodiments. **Figure14A** shows an exploded view of introducer 20 and sensor probe 60 before assembly. Accordingly, introducer cap 21 is connected to the introducer 20 at one end (e.g., proximal end) and is comprised of introducer cap snap 211 and introducer cap snap holder 212. The introducer cap snap 211 can be configured to couple with the hammer (e.g., **Figures 2 - 6**) and drives introducer 20 during insertion. **Figure 14B** shows a spatial view of introducer 20, introducer cap 21, sensor probe 60, and contacts plate 61, according to some embodiments, and a magnified view of the introducer tip 22 and sensor probe tip 65 before sensor probe 60 insertion.

**Figure 14C** shows a spatial view of introducer 20 and introducer cap 21 before sensor probe insertion, according to some embodiments. **Figure 14D** shows a spatial view of introducer 20, introducer cap 21, sensor probe 60, and contacts plate 61, and a magnified view of the introducer tip 22 and sensor probe tip 65 after sensor probe 60 insertion within the subcutaneous tissue and before retraction of introducer 20 (end of the first mounting phase), according to some embodiments.

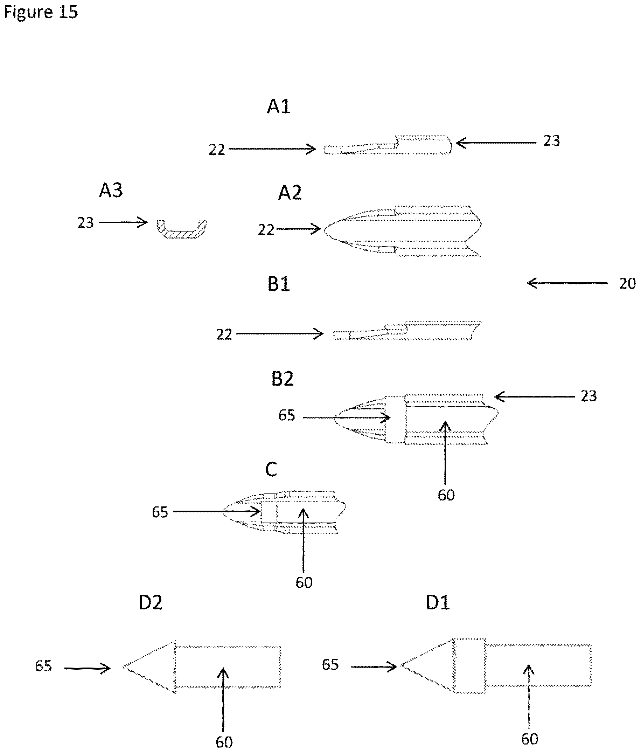

**Figure 15** shows the introducer tip 22 and sensor tip 65, according to some embodiments. Figures 15A1-3 show side view **(15A1)**, top view **(15A2)**, and cross-sectional view **(15A3)** of introducer tip 22, according to some embodiments. Introducer 20, in some embodiments, includes a U-shape cross section having at least one and preferably two longitudinal protrusions: sensor probe holders 23, configured to support sensor probe 60 when it is coupled with introducer 20. Figures 15B1-2 show a side view **(15B1)** and a top view **(15B2)** of introducer tip 22 and sensor probe tip 60. In some embodiments, sensor tip 65 has a rectangular shape. Figure 15C shows a top view of a sharp tip 22 of the introducer, and sensor tip 65 of probe 60 (here, sensor tip 65 has a square shape). Figures 15D1 and 15D2 show schemes, according to some embodiments, of the senor probe tip having a sharp end.

Figures 16A - C and 16A1 - C1 show spatial **(16A-C)** and magnified **(16A1-C1)** views, of the sensor 50, sensor probe 60, introducer tip 22, and sensor tip 65, according to some embodiments, during a first mounting phase and a second mounting phase. Since it is desired to minimize traumatic injury at the tissue surrounding the sensor probe tip and minimize the consequent inflammatory reaction because inflammation reduces the electrodes sensitivity to glucose, in the first mounting phase, the coupled introducer 20 and sensor probe 60 are concomitantly inserted into the subcutaneous tissue. In some embodiments, at the end of forward/first direction displacement of introducer 20 and sensor probe 60, sensor probe 60 is further displaced (e.g., 1-3mm) in the same (first/forward) direction (i.e., there is a relative movement between introducer 20 and sensor probe 60). Thus, at the end of the first mounting phase, in some embodiments, sensor probe tip 65 is located distally to the introducer tip 22. The sensor probe 60 is relatively thin 50-100 microns such that and movement within the subcutaneous tissue causes minimal trauma. Figures 16A - A1 show sensor probe 60 coupled with introducer 20 before insertion - sensor probe tip 65 resides within the introducer 20. Figures 16B - B1 show the introducer tip 22 and the sensor probe tip 65 at the end of the first mounting phase, with the sensor probe tip 65 protruding from the introducer tip 22 (e.g., positioned 1-3mm apart). **Figures 14C** - C1 show sensor 50 and probe 60 after removal of introducer 20 (not shown) (end of second mounting phase).

**Figures 17** and **18** show an assembly process of mounting assembly 1, according to some embodiments. In a first assembly stage, probe compartment 6 is assembled and then sterilized (e.g., gamma, e-beam radiation). The probe compartment 6 is then sealed and provides protection to sensor probe 60 against biologic and chemical contamination. In some embodiments, probe compartment 6 is comprised of at least a portion of mounting assembly 1 and at least a portion of sensor 50 (e.g., **Figures 1 - 3**). Following sterilization of the probe compartment 6, the non-sterilized spring compartment 9 can be assembled adjacent (e.g., on-top) of sterilized probe compartment 6 (e.g., in a clean room). The spring compartment 9, in some embodiments, comprises at least a portion of mounting assembly 1 and at least a portion of sensor 50 (see e.g., **Figures 1- 3**). The introducer 20, according to some embodiments, spans mounting assembly 1 and is configured to be received in both probe compartment 6 and spring compartment 9.

Figures 17A-B show exploded view **(17A)** and oblique cross section view **(17B)** of the sterilized, sealed compartment (probe compartment 6), according to some embodiments. The probe compartment 6 can include a plurality of, and in some embodiments, all of (parts stacked, e.g., from the bottom-up) protecting lid 17, protecting lid seal 174, probe compartment seal 162, probe compartment housing 16, adhesive 56, sensor housing lower cover 55, sensor base plate, 54, introducer 20, base plate seal 546, sensor probe 60, and contacts plate 61.

Figures 18A1 - F1 and 18A2 - F2 show spatial views **(18A1** - **F1**) and oblique views **(18A2 - F2)**, respectively, of the various assembly stages of the mounting assembly 1, according to some embodiments. Figures 18A1-2 show sterile probe compartment 6 after assembly (e.g., assembly process shown in **Figure 17**) and sterilization. The sterile probe/compartment (probe compartment 6) can include a plurality of, and in some embodiments, all of protecting lid 17, probe compartment housing 16, sensor base plate 54, and introducer 20. Figures 18B1-2 show the assembled PCBA 53, according to some embodiments, Figures 18C1-2 show the assembled sensor housing upper cover 51, according to some embodiments, and Figures 18D1-2 show the assembled spring compartment base 15 and the retraction spring 32, according to some embodiments. Figures 18E1-2 show the assembled introducer cap 21, according to some embodiments, and Figures 18F1-2 show spring compartment housing 19 and insertion spring loaded mechanism (operating button 11, other component are shown in **Figures 2-6**), according to some embodiments.

At a later stage of assembly, according to some embodiments, spring compartment housing 19 can be rigidly connected to probe compartment housing 16 forming mounting assembly 1 (as shown in **Figures 1 - 6**). In some examples, which are not claimed, assembled mounting assembly 1 can be sterilized with a gas (e.g., ethylene oxide), and sealed probe compartment 6, which can be pre-sterilized with radiation (e.g., gamma or e-beam), protects the gas sensitive enzyme (deposits on the probe 60) from potential damage of the gas. In such a configuration, both compartments (probe compartment 6 and spring compartment 9) can be sterilized as well as all components of the sensor 50 (including PCBA).

While various examples have been described and illustrated herein, those of ordinary skill in the art will readily envision a variety of other means and/or structures for performing the function, and/or obtaining the results and/or one or more of the objects/advantages described herein, and each of such variations and/or modifications is deemed to be described herein. More generally, those skilled in the art will readily appreciate that all parameters, dimensions, materials, steps, and configurations described herein are meant to be merely an example and that the actual parameters, dimensions, materials, steps, and configurations will depend upon the specific application or applications for which the teachings is/are used. Those skilled in the art will recognize, or be able to ascertain using no more than routine experimentation, many equivalents to the specific examples described herein. It is therefore to be understood that the foregoing is presented by way of example only. Examples of the present disclosure are directed to each individual feature, device, system, article, material, kit, step, function/functionality, and method described herein. In addition, any combination of two or more such features, devices, systems, articles, materials, kits, steps, functions/functionality, and methods, if such features, systems, articles, materials, kits, steps, functions/functionality, and methods are not mutually inconsistent, is included.

Examples 2. disclosed herein may also be combined with one or more features, as well as complete systems, devices, and/or methods, to yield yet other examples.

The acts performed as part of the method(s) may be ordered in any suitable way. Accordingly, examples may be constructed in which acts are performed in an order different than illustrated, which may include performing some acts simultaneously, even though shown as sequential acts.

The indefinite articles "a" and "an," as used herein in the specification and in the claims, unless clearly indicated to the contrary, should be understood to mean "at least one."

The terms "can" and "may" are used interchangeably in the present disclosure, and indicate that the referred to element, component, structure, function, functionality, objective, advantage, operation, step, process, apparatus, system, device, result, or clarification, has the ability to be used, included, or produced, or otherwise stand for the proposition indicated in the statement for which the term is used (or referred to).

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of" "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of and "consisting essentially of' shall be closed or semi-closed transitional phrases, respectively.

The invention is defined in the appended claims.

## Claims

1. A continuous glucose monitoring system for continuously monitoring glucose levels of a user, the system comprising:
a mounting assembly (1) including a first, non-sealed, non-sterilized compartment (9) and a second sealed, sterilized compartment (6);
a sensing device comprising a sensor and a sensor probe (60), and
an introducer (20) configured to insert the sensor probe (60) into subcutaneous tissue of the user, wherein:
at least a first portion of the sensing device is housed within the first, non-sealed, non-sterilized compartment (9), and at least a second portion of the sensing device is housed within the second, sealed, sterilized compartment (6) prior to mounting the sensing device on the user, and
the first, non-sealed, non-sterilized compartment (9) includes an insertion means comprising a spring-loaded driving mechanism configured to mount the sensing device onto the user.

2. The system of claim 1, wherein the sensor includes a sensor housing.

3. The system of claim 1 or claim 2, wherein the sensor includes a sensor control unit.

4. The system of claim 2, wherein the sensor probe includes a first portion configured for insertion into subcutaneous tissue, and a second portion configured for receiving by the sensor housing.

5. The system of any of claims 2 or 4, wherein the sensor housing comprises an upper cover and a lower cover.

6. The system of any of claims 1-5, wherein the sensor includes a printed circuit board assembly (PCBA).

7. The system of any of claims 2, 4 or 6 wherein at least one of the sensor housing and the PCBA also includes at least one of a receiver, a transmitter, a processing circuit, a battery, an alarm system, and data storage means.

8. The system of any of claims 1-7, wherein the sensor probe includes at least of: at least one working electrode, a counter electrode, at least two (2) electrical conductors, and at least two (2) contact pads.

9. The system of any of claims 1-8, wherein the sensor probe includes at least one working electrode, a counter electrode, at least two (2) electrical conductors, and at least two (2) contact pads.

10. The system of any of claims 1-9, wherein the sensor probe is configured with a planar shape, and includes at least two (2) electrodes.

11. The system of claim 10, wherein the at least two (2) electrodes comprise at least a working electrode and a counter electrode, wherein the working electrode is configured for positioning on at least one of a first side of the sensor probe and at a distal end thereof, and the counter electrode is configured for arrangement on a side of the sensor probe opposite to the side where the working electrode is positioned on the sensor probe.

12. The system of claim 10 or claim 11, wherein each electrode is connected via electrical conductors to a respective contact pad.

13. The system of claim 12, wherein one or more contact pads are configured for:
positioning on at least one side of a contacts plate, the contacts plate optionally included with the sensor probe, or
positioning on each side of a contacts plate, the contacts plate optionally included with the sensor probe.

14. The system of claim 13, wherein the contacts plate is positioned approximately perpendicular to the sensor probe.

15. The system of claim 14, wherein:
the contacts plate is positioned within the sensor housing,
upon the contacts plate being positioned within the sensor housing, at least one (1) contact pad is facing the PCBA, and optionally, another contact pad is facing an opposite direction,
and/or
the contacts plate is folded on a first side at 180 degrees, such that each contact pad is facing the PCBA.

## Patentansprüche

1. Kontinuierliches Glucoseüberwachungssystem zum kontinuierlichen Überwachen der Glucosespiegel eines Benutzers, wobei das System umfasst:
eine Anbringanordnung (1), die ein erstes nichtversiegeltes, nicht-sterilisiertes Fach (9) und ein zweites versiegeltes, sterilisiertes Fach (6) einschließt;
eine Abfühlvorrichtung, die einen Sensor und eine Sensorsonde (60) umfasst, und
eine Einführhilfe (20), die ausgelegt ist, um die Sensorsonde (60) in Subkutangewebe des Benutzers einzuführen, wobei:
mindestens ein erster Anteil der Abfühlvorrichtung in dem ersten nicht-versiegelten, nicht-sterilisierten Fach (9) untergebracht ist, und mindestens ein zweiter Anteil der Abfühlvorrichtung in dem zweiten versiegelten, sterilisierten Fach (6) untergebracht ist, bevor die Abfühlvorrichtung an dem Benutzer angebracht wird, und
das erste nicht-versiegelte, nicht-sterilisierte Fach (9) ein Einführmittel einschließt, das einen federgestützten Antriebsmechanismus umfasst, der ausgelegt ist, um die Abfühlvorrichtung an dem Benutzer anzubringen.

2. System nach Anspruch 1, wobei der Sensor ein Sensorgehäuse einschließt.

3. System nach Anspruch 1 oder Anspruch 2, wobei der Sensor eine Sensorsteuereinheit einschließt.

4. System nach Anspruch 2, wobei die Sensorsonde einen ersten Anteil, der zum Einführen in Subkutangewebe ausgelegt ist, und einen zweiten Anteil, der ausgelegt ist, um von dem Sensorgehäuse aufgenommen zu werden, einschließt.

5. System nach einem der Ansprüche 2 oder 4, wobei das Sensorgehäuse eine obere Abdeckung und eine untere Abdeckung umfasst.

6. System nach einem der Ansprüche 1-5, wobei der Sensor eine gedruckte Schaltplatinenanordnung (PCBA) einschließt.

7. System nach einem der Ansprüche 2, 4 oder 6, wobei mindestens eines von dem Sensorgehäuse und der PCBA auch mindestens eines von einem Empfänger, einem Sender, einer Verarbeitungsschaltung, einer Batterie, einem Alarmsystem und Datenspeichermittel(n) einschließt.

8. System nach einem der Ansprüche 1-7, wobei die Sensorsonde mindestens eines von: mindestens einer Arbeitselektrode, einer Gegenelektrode, mindestens zwei (2) elektrischen Leitern und mindestens zwei (2) Kontaktstellen einschließt.

9. System nach einem der Ansprüche 1-8, wobei die Sensorsonde mindestens eine Arbeitselektrode, eine Gegenelektrode, mindestens zwei (2) elektrische Leiter und mindestens zwei (2) Kontaktstellen einschließt.

10. System nach einem der Ansprüche 1-9, wobei die Sensorsonde mit einer planaren Form ausgelegt ist und mindestens zwei (2) Elektroden einschließt.

11. System nach Anspruch 10, wobei die mindestens zwei (2) Elektroden mindestens eine Arbeitselektrode und eine Gegenelektrode umfassen, wobei die Arbeitselektrode zum Positionieren auf mindestens einem von einer ersten Seite der Sensorsonde und einem distalen Ende davon ausgelegt ist, und die Gegenelektrode zur Anordnung auf einer Seite der Sensorsonde ausgelegt ist, die der Seite gegenüber liegt, wo die Arbeitselektrode auf der Sensorsonde positioniert ist.

12. System nach Anspruch 10 oder Anspruch 11, wobei jede Elektrode über elektrische Leiter mit einer jeweiligen Kontaktstelle verbunden ist.

13. System nach Anspruch 12, wobei eine oder mehrere Kontaktstellen ausgelegt ist/sind zum:
Positionieren auf mindestens einer Seite einer Kontaktplatte, wobei die Kontaktplatte gegebenenfalls in die Sensorsonde eingeschlossen ist, oder
Positionieren auf jeder Seite einer Kontaktplatte, wobei die Kontaktplatte gegebenenfalls in die Sensorsonde eingeschlossen ist.

14. System nach Anspruch 13, wobei die Kontaktplatte ungefähr rechtwinklig zu der Sensorsonde positioniert ist.

15. System nach Anspruch 14, wobei:
die Kontaktplatte innerhalb des Sensorgehäuses positioniert ist,
wenn die Kontaktplatte innerhalb des Sensorgehäuses positioniert ist, mindestens eine (1) Kontaktstelle zu der PCBA weist, und gegebenenfalls eine andere Kontaktstelle in eine Gegenrichtung weist,
und/oder
die Kontaktplatte auf einer ersten Seite um 180 Grad gefaltet ist, so dass jede Kontaktstelle zu der PCBA weist.

## Revendications

1. Système de surveillance de glucose en continu pour surveiller en continu les niveaux de glucose d'un utilisateur, le système comprenant :
un ensemble de montage (1) comprenant un premier compartiment non-étanchéifié, non stérilisé (9) et un second compartiment étanche, stérilisé (6) ;
un dispositif de détection comprenant un capteur et une sonde de capteur (60), et
un dispositif d'insertion (20) configuré pour insérer la sonde de capteur (60) dans le tissu sous-cutané de l'utilisateur :
au moins une première partie du dispositif de détection étant logée dans le premier compartiment non-étanchéifié, non stérilisé (9), et au moins une seconde partie du dispositif de détection étant logée dans le second compartiment étanche, stérilisé (6) avant le montage du dispositif de détection sur l'utilisateur, et
le premier compartiment non-étanchéifié, non stérilisé (9) comprenant un moyen d'insertion comprenant un mécanisme d'entraînement à ressort configuré pour monter le dispositif de détection sur l'utilisateur.

2. Système selon la revendication 1, le capteur comprenant un boîtier de capteur.

3. Système selon la revendication 1 ou 2, le capteur comprenant une unité de commande de capteur.

4. Système selon la revendication 2, la sonde de capteur comprenant une première partie configurée pour être insérée dans le tissu sous-cutané, et une deuxième partie configurée pour être reçue par le boîtier de capteur.

5. Système selon l'une quelconque des revendications 2 ou 4, le boîtier de capteur comprenant un couvercle supérieur et un couvercle inférieur.

6. Système selon l'une quelconque des revendications 1 à 5, le capteur comprenant un ensemble carte de circuit imprimé (PCBA).

7. Système selon l'une quelconque des revendications 2, 4 ou 6, au moins l'un parmi le boîtier de capteur et la PCBA comprenant également au moins l'un parmi un récepteur, un émetteur, un circuit de traitement, une batterie, un système d'alarme et des moyens de stockage de données.

8. Système selon l'une quelconque des revendications 1 à 7, la sonde de capteur comprenant au moins l'un de : au moins une électrode de travail, une contre-électrode, au moins deux (2) conducteurs électriques, et au moins deux (2) plots de contact.

9. Système selon l'une quelconque des revendications 1 à 8, la sonde de capteur comprenant au moins une électrode de travail, une contre-électrode, au moins deux (2) conducteurs électriques, et au moins deux (2) plots de contact.

10. Système selon l'une quelconque des revendications 1 à 9, la sonde de capteur étant configurée avec une forme plane, et comprenant au moins deux (2) électrodes.

11. Système selon la revendication 10, les au moins deux (2) électrodes comprenant au moins une électrode de travail et une contre-électrode, l'électrode de travail étant configurée pour être positionnée sur au moins l'un d'un premier côté de la sonde de capteur et à une extrémité distale de celle-ci, et la contre-électrode étant configurée pour être disposée sur un côté de la sonde de capteur opposé au côté où l'électrode de travail est positionnée sur la sonde de capteur.

12. Système selon la revendication 10 ou 11, chaque électrode étant connectée par l'intermédiaire des conducteurs électriques à un plot de contact respectif.

13. Système selon la revendication 12, un ou plusieurs plots de contact étant configurés pour :
le positionnement sur au moins un côté d'une plaque de contacts, la plaque de contacts étant éventuellement incluse avec la sonde de capteur, ou
le positionnement sur chaque côté d'une plaque de contacts, la plaque de contacts étant éventuellement comprise avec la sonde de capteur.

14. Système selon la revendication 13, la plaque de contacts étant positionnée approximativement perpendiculairement à la sonde de capteur.

15. Système selon la revendication 14,
la plaque de contacts étant positionnée à l'intérieur du boîtier de capteur,
lorsque la plaque de contacts est positionnée l'intérieur du boîtier de capteur, au moins un (1) plot de contact étant orienté vers la PCBA, et éventuellement, un autre plot de contact étant orienté vers une direction opposée, et/ou
la plaque de contacts étant pliée sur un premier côté à 180 degrés, de sorte que chaque plot de contact fasse face à la PCBA.
